(19) Europäisches Patentamt
European Patent Office
Office européen des brevets

(11) EP 0 884 321 B1

(12) **EUROPEAN PATENT SPECIFICATION**

(45) Date of publication and mention of the grant of the patent:
**19.02.2003 Bulletin 2003/08**

(51) Int Cl.⁷: **C07F 9/655**, A23L 3/3544, A23L 3/3553, A61K 31/665, A61K 7/00, A23K 3/00

(21) Application number: **98110676.8**

(22) Date of filing: **10.06.1998**

(54) **Ascorbic acid 2-phosphate zinc salt and process for manufacturing the same**

Ascorbinsäure 2-phosphatzinksalz und Verfahren zu seiner Herstellung

Sel de zinc du phosphate de l'acide ascorbique et son procédé de préparation

(84) Designated Contracting States:
**AT BE CH DE DK ES FI FR GB GR IE IT LI LU MC NL PT SE**

(30) Priority: **11.06.1997 JP 15397297**

(43) Date of publication of application:
**16.12.1998 Bulletin 1998/51**

(73) Proprietor: **SHOWA DENKO KABUSHIKI KAISHA**
**Minato-ku, Tokyo (JP)**

(72) Inventors:
- **Suzuki, Masahiro**
  **Sanbu-gun, Chiba, 299-3234 (JP)**
- **Tsuzuki, Toshi, c/o Showa Denko K.K.**
  **Chiba-shi, Chiba, 267-0056 (JP)**
- **Itoh, Shinobu**
  **Minato-ku, Tokyo, 105-8518 (JP)**
- **Ogata, Eiji**
  **Minato-ku, Tokyo, 105-8518 (JP)**

(74) Representative: **Strehl Schübel-Hopf & Partner**
**Maximilianstrasse 54**
**80538 München (DE)**

(56) References cited:
**FR-A- 1 489 249** **US-A- 3 671 549**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 884 321 B1

## Description

### FIELD OF THE INVENTION

The present invention relates to a novel L-ascorbic acid 2-phosphate salt, a process for manufacturing the salt and a method of use thereof. More specifically, the present invention relates to L-ascorbic acid 2-phosphate zinc salt or a salt hydrate of zinc L-ascorbic acid 2-phosphate (hereinafter, unless otherwise indicated, the salt including the salt hydrate thereof is referred to as an "L-ascorbic acid 2-phosphate zinc salt") which is a soluble L-ascorbic acid 2-phosphate salt having improved stability. L-ascorbic acid 2-phosphate zinc salt does not cause an extreme alkaline condition on dissolving in water and also exhibits antimicrobial activity. The present invention also relates to a method for manufacturing the salt and a method of use thereof. By using this salt, an L-ascorbic acid 2-phosphate-containing preparation (e.g., a cosmetic preparation, medical preparation, agricultural chemical preparation, animal drug preparation, foodstuff preparation and feedstuff preparation) having improved solubility, stability and antimicrobial properties (including antiseptic, deodorizing and antidandruff effects) and which is stable under weakly acidic conditions, can be obtained.

### BACKGROUND OF THE INVENTION

L-Ascorbic acid (vitamin C) imparts many physiological effects. For example, vitamin C restrains lipid peroxide, accelerates the formation of collagen, retards the formation of melanin and enhances immune functions, and has hitherto been used to achieve these effects in the fields of medicine, agricultural chemicals, animal drugs, foodstuffs, feedstuffs and cosmetic preparations. Many preparations containing L-ascorbic acid are commercially available. However, L-ascorbic acid has poor aging stability, and the effect of vitamin C cannot be always obtained even if such a preparation is used.
In order to improve stability, derivatives obtained by converting the hydroxyl groups in the enediol moiety at the 2-or 3-position each into a glycosyl group (for example, EP-A-0 539 196 (JP-A-5-117290) (the term "JP-A" as used herein means an "unexamined published Japanese patent application")) or into a phosphoric acid ester (for example, GB-A-1 201 404 (JP-B-52-18191) (the term "JP-B" as used herein means an "examined Japanese patent publication") and US-A-5 110 951 (JP-A-2-279690)) have been proposed.
However, L-ascorbic acid 2-glycoside salts are converted into L-ascorbic acid in vivo (particularly in humans) at a low rate as compared with L-ascorbic acid 2-phosphates. Therefore, a prompt effect cannot be achieved and an adequately high vitamin C effect can hardly be obtained.
FR-A-1 489 249 discloses the preparation of metal salts of L-ascorbic acid phosphate.
US-A-3 671 549 discloses a process for the preparation of alkali or alkaline earth metal salts of ascorbyl-2-phosphate.
L-Ascorbic acid 2-phosphates provide a prompt effect, and an adequately high vitamin C effect can be easily obtained. However, conventionally known L-ascorbic acid 2-phosphates are a magnesium salt, a calcium salt and a sodium salt, and among these salts, L-ascorbic acid 2-phosphate magnesium salt having excellent stability does not have sufficiently high solubility in water and is difficult to form into an aqueous solution having a concentration of 30% or more. The calcium salt scarcely dissolves in water.
Sodium L-ascorbic acid 2-phosphate having satisfactorily improved solubility can be easily formed into an aqueous solution having a concentration of about 30%, but does not have a stability that is comparable with that of the magnesium salt. Furthermore, when dissolving any of these salts, the pH of the resulting solution is in an alkaline region of approximately from 8 to 10. When the pH is adjusted to a weakly acidic condition, the stability is disadvantageously lowered.
Although the above-described L-ascorbic acid derivatives have been proposed, an L-ascorbic acid 2-phosphate having excellent solubility and which is sufficiently stable even under weakly acidic conditions has not yet been obtained.
In obtaining a salt in general, neutralization of an acid or base is used in many cases. However, this method is not appropriate for manufacturing an L-ascorbic acid 2-phosphate salt on an industrial scale. This is because an L-ascorbic acid 2-phosphate solution is strongly acidic and unstable.

### SUMMARY OF THE INVENTION

It is therefore an object of the present invention to provide an L-ascorbic acid 2-phosphate having excellent solubility and which exhibits sufficiently improved stability even under weakly acidic conditions.
Under these circumstances, the present inventors have conducted extensive investigations and as a result, have found that hitherto unknown L-ascorbic acid 2-phosphate zinc salt is a compound having highly improved solubility

and stability even under weakly acidic conditions, and also provides an antimicrobial (antiseptic) effect.

The above object of the present invention has been achieved by providing L-ascorbic acid 2-phosphate zinc salt and a salt hydrate of zinc L-ascorbic acid 2-phosphate which, in addition to the above properties, has also been found to have an antimicrobial effect. The present invention further provides a process for manufacturing the L-ascorbic acid 2-phosphate zinc salt and salt hydrate thereof and a method of using the salt and salt hydrate thereof in cosmetic and medical preparations, agricultural chemical preparations, animal drug preparations, foodstuff preparations and feedstuff preparations so as to increase the action of vitamin C or to provide an antimicrobial, antiseptic, deodorizing or antidandruff effect.

DESCRIPTION OF THE PREFERRED EMBODIMENTS

Examples of the process for manufacturing the compound of the present invention include a manufacturing process starting from a soluble L-ascorbic acid 2-phosphate salt and using an ion exchange technique or the like, and a combination of this manufacturing process with other purification methods, as needed. However, the present invention is by no means limited thereto.

If L-ascorbic acid 2-phosphoric acid is represented by $ApH_3$, the zinc salt of the present invention is represented by $Ap_2Zn_3$ and has a structure as shown in formula (1) below:

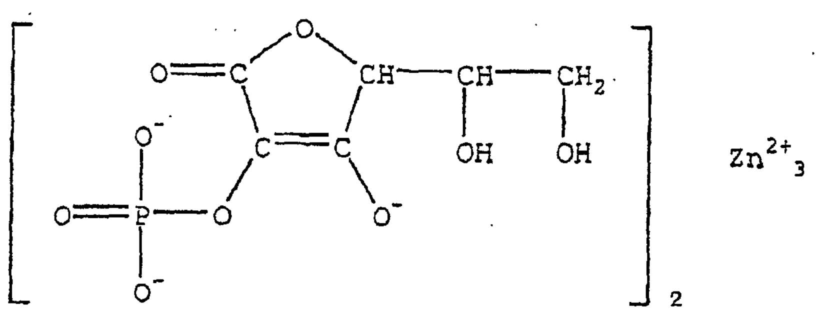

(1)

$Ap_2Zn_3$ is white powder and can be obtained as an anhydride or salt hydrate. The salt hydrate is considered to be a hydrate $Ap_2Zn_3 \cdot nH_2O$ of $Ap_2Zn_3$.

In a preferred embodiment of the L-ascorbic acid 2-phosphate zinc salt or salt hydrate thereof of the present invention, 95 mol% or more of the entire cationic charge constitutes zinc ion and 95 mol% or more of the entire anionic charge constitutes L-ascorbic acid 2-phosphate ion. The Zn cation and Ap anion are present in sufficient amounts if each is contained in total in the above-described amounts. For example, when $Ap_2Zn_3$ is present in an amount of 95 mol% or more of the total L-ascorbic acid 2-phasphate salt content, $Ap_2Zn_2Mg$ or the like may be present in part. However, this is acceptable if the zinc ion content is 95 mol% or more of the total cation content.

The L-ascorbic acid 2-phosphate zinc salt of the present invention may be manufactured by a process using, for example, a soluble L-ascorbic acid 2-phosphate salt other than the zinc salt, such as $Ap_2Mg_3$, as a starting material and replacing the magnesium with zinc by chemical reaction or using an ion exchange technique (e.g., treating with an ion exchange resin). This process may also be combined with a purification method. Examples of the chemical reaction include:

$$Ap_2Mg_3 + 3ZnCl_2 \rightarrow Ap_2Zn_3 + 3MgCl_2$$

In a manufacturing process using an ion exchange technique, the soluble L-ascorbic acid 2-phosphate salt used as a starting material is not necessarily purified, but may be, for example, an L-ascorbic acid 2-phosphate-containing solution obtained by reacting L-ascorbic acid with phosphorus oxychloride. The ion exchange step may employ, for example, cation exchange column chromatography or anion exchange chromatography.

When using cation exchange column chromatography, an aqueous L-ascorbic acid 2-phosphate zinc salt solution may be eluted by passing an L-ascorbic acid 2-phosphate solution through a column containing a zinc type cation exchange resin (preferably a strong cationic exchange resin).

In the case of using anion exchange chromatography, a solution containing L-ascorbic acid 2-phosphate is passed through the anion exchange resin column to adsorb L-ascorbic acid 2-phosphate ion onto the resin, a zinc salt solution is passed therethrough, and the L-ascorbic acid 2-phosphate zinc salt fraction is collected.

In the case where the L-ascorbic acid 2-phosphate solution that is added to the column contains a substance

which forms a zinc salt having low solubility, the solution is previously processed to remove such substances (for example, in the case of phosphorus ion, a zinc salt or magnesium salt is added to precipitate and remove the substance). In this manner, the column chromatography can be performed more efficiently.

The chromatography conditions (temperature, flow rate, composition of added solution, concentration, amount of solution, etc.) may be established by taking into account general column chromatography parameters (e.g., column size, physical properties of the resin, exchange capacity, separation capability, viscosity of the added solution, solubility, etc.) well known to those of ordinary skill in the art.

L-ascorbic acid 2-phosphate zinc salt powder can be obtained directly from the above solution containing L-ascorbic acid 2-phosphate zinc salt, or by precipitating or the like in an organic solvent and removing the solvent by spray drying, vacuum drying or freeze drying. When the L-ascorbic acid 2-phosphate zinc salt powder is obtained by precipitating in an organic solvent, the zinc salt added to the column in the anion exchange method preferably, in view of purity, is a salt which is highly soluble in the organic solvent (for example, in the case of using acetone as the organic solvent, zinc chloride or zinc propionate may be used).

The thus-obtained L-ascorbic acid 2-phosphate zinc salt has excellent solubility and is easily formed into an aqueous solution having a concentration of 50 wt% or more. Furthermore, the L-ascorbic acid 2-phosphate zinc salt of the present invention is remarkably stable even under weakly acidic conditions, and a composition containing an L-ascorbic acid 2-phosphate having sufficiently improved stability even in such a pH region can be obtained. Furthermore, a composition also having an antimicrobial effect (including antiseptic, deodorizing and antidandruff effects) can be obtained. Furthermore, vitamin C can be effectively supplied by administering the composition to humans or animals. Examples of the composition include cosmetic preparations, medical preparations, agricultural chemical preparations, animal drug preparations, foodstuff preparations and feedstuff preparations. The ingredients of the composition and the amount of the L-ascorbic acid 2-phosphate zinc salt added thereto are the same as in the case of using a conventional L-ascorbic acid 2-phosphate salt.

For example, a general and preferred range for the content (wt%) of the L-ascorbic acid 2-phosphate zinc salt when added to a cosmetic preparation, a medical preparation, an agricultural chemical preparation, an animal drug preparation, a foodstuff preparation and a feedstuff preparation may be as follows:

| Preparation Type | General | Preferred |
|---|---|---|
| Cosmetic preparation | 0.01 - 50% | 0.1 - 5% |
| Medical preparation | 0.01 - 50% | 0.1 - 20% |
| Agricultural chemical preparation | 0.01 - 50% | 0.1 - 10% |
| Animal drug preparation | 0.01 - 50% | 0.1 - 20% |
| Foodstuff preparation | 0.001 - 50% | 0.002 - 5% |
| Feedstuff preparation | 0.001 - 50% | 0.002 - 5% |

In the case of a salt hydrate, the above amounts are increased to take into account the water of hydration.

EXAMPLES

The present invention is described in greater detail below by reference to the following Examples, however, the present invention should not be construed as being limited thereto.

In the Examples and Test Examples, the analysis of an L-ascorbic acid 2-phosphate salt or an ion thereof was performed using an Ap-containing solution having a known concentration (prepared from L-ascorbic acid 2-phosphate magnesium salt) as a standard by an HPLC method using a Shodex IEC DEAE-825 resin (ammonium acetate 10 mM $\rightarrow$ 1M gradient, detected spectrophotometrically at 265 nm). The elemental analysis was performed by an ICP emission method, flame photometry and an ion chromatography method. Unless otherwise indicated, the reagents used herein were commercially available reagents.

Example 1

Under a nitrogen atmosphere, 10 g of L-ascorbic acid was dissolved in a mixed solvent of 135 ml of water and 15 g of pyridine, and the resulting mixed solution was cooled to 0°C and adjusted to a pH of 11.5 with a 12% (% by weight, hereinafter the same) potassium hydroxide solution. While keeping the solution at a temperature of from 0 to 5°C, 15 g of phosphorus oxychloride was added by means of a quantitative pump. During the addition, the pH was kept within a range of from 11.45 to 11.55 with a 12% potassium hydroxide solution. After completing the addition, the mixture was stirred at the same temperature for 10 minutes, and 70 ml of a 1.5M magnesium chloride solution was

gradually added thereto. The resulting solution was further stirred for 30 minutes, and the precipitate was then removed by suction filtration.

The solution thus obtained was added to a column containing an OH-type anion exchange resin Diaion SA 10A (trade name, manufactured by Mitsubishi Kasei KK) having a column size of 2.5 cmϕ x 50 cm, and washed with 1 L of water. The running direction of the solution was reversed, 250 ml of a 3M zinc chloride solution was added at a rate of 10 ml/min, and 250 ml of an eluent was obtained. To the eluent, 1 L of acetone was gradually added, and the mixture was stirred for 30 minutes. The precipitate was collected by suction filtration, washed three times with 200 ml of acetone, and vacuum dried to obtain 4.3 g of powder. A small portion of the powder was dissolved in purified water in an amount of 100 ppm and analyzed by the HPLC method described above. As a result, the solution was found to contain Ap in a concentration of 56.7 ppm. Namely, the powder contained Ap in an amount of 56.7%. The elemental analysis for the powder was as follows. C: 16.2%, H: 3.6%, O: 50.2%, P: 7.0%, Zn: 22.3%, Mg: 0.1%, Cl: 0.4%, and K: <0.1%. Thus, the powder was verified to have a purity of 95% or more as $Ap_2Zn_3 \cdot 10H_2O$.

Example 2

To a cation exchange column containing the resin Diaion SK 1B (trade name, manufactured by Mitsubishi Kagaku KK) having a column size of 5 cmϕ × 25 cm, 1.5 L of 1M sulfuric acid, 0.5 L of water, 1L of 1M zinc sulfate and 2 L of water were added in sequence at a rate of 20 ml/min to form a zinc type resin.

To the column, 0.5 L of a 10% aqueous solution of L-ascorbic acid PM (L-ascorbic acid 2-phosphate magnesium salt, trade name, produced by Showa Denko KK) and 0.5 L of water were added in sequence at a rate of 10 ml/min, and 1 L of eluent was collected. The eluent thus collected was freeze dried to obtain 52 g of powder. A small portion of this powder was dissolved in purified water in an amount of 100 ppm, and the resulting solution was analyzed by the HPLC method described above. As a result, the solution was found to contain Ap in a concentration of 58.6 ppm. Namely, the powder obtained contained Ap in an amount of 58.6%. The elemental analysis for the powder was as follows. C: 16.7%, H: 3.5%, O: 50.1%, P: 7.2%, Zn: 22.5%, and Mg: <0.1%. Thus, the powder was verified to have a purity of 99% or more as $Ap_2Zn_3$-$9H_2O$.

Unless otherwise indicated, in Test Examples 1 and 2 and in Example 3 below, the L-ascorbic acid 2-phosphate zinc salt obtained in Examples 1 and 2 was used in the following evaluations.

Test Example 1 — Stability

The stability of L-ascorbic acid 2-phosphate zinc salt was compared to that of L-ascorbic acid 2-phosphate magnesium salt. Each salt was dissolved in a buffer solution (50 mM acetic acid-NaOH, pH: 5, or 50 mM Tris-HCl, pH: 8) to a concentration of about 0.1% and stored at 50°C for 476 hours. The concentrations of the L-ascorbic acid 2-phosphate before and after storage were analyzed by the HPLC method.

Table 1

| L-ascorbic acid 2-phosphate | Stability (100% = concentration before storage) | |
|---|---|---|
| | pH: 5 | pH: 8 |
| L-ascorbic acid 2-phosphate zinc salt (Example 1) | 91 | 93 |
| L-ascorbic acid 2-phosphate zinc salt (Example 2) | 92 | 95 |
| L-ascorbic acid 2-phosphate magnesium salt | 82 | 95 |

The above results show that the stability of the L-ascorbic acid 2-phosphate zinc salt of the present invention is hardly reduced even when stored under weakly acidic conditions.

Test Example 2 — Antimicrobial effect

To NUTRIENT BROTH (produced by DIFCO), 0.1% of an L-ascorbic acid 2-phosphate salt was added thereto, and the pH was adjusted to 6.8 using hydrochloric acid or sodium hydroxide. Using 5 ml of the medium thus prepared, test bacterium were inoculated in a concentration of about $10^7$ cells/ml and incubated in a test tube at 37°C for 24 hours. The results are shown in Table 2 below.

In Table 2, "+" indicates that turbidity increased after incubation, and "-" indicates no increase in turbidity. As a control, the same test was performed using a medium not containing an L-ascorbic acid 2-phosphate salt.

Table 2

| L-Ascorbic acid 2-phosphate | Test bacterium | |
|---|---|---|
| | *E. coli* (K-12 strain) | *B. subtilis* (168 strain) |
| Zinc salt (Example 1) | - | - |
| Zinc salt (Example 2) | - | - |
| Magnesium salt | + | + |
| Sodium salt | + | + |
| Not added | + | + |

The above results show that the L-ascorbic acid 2-phosphate zinc salt of the present invention has antimicrobial properties.

Example 3 — Cosmetic Preparation

One example of a cosmetic preparation in accordance with the present invention is shown below.

Table 3

| | Ingredient | % by weight |
|---|---|---|
| (A) | Liquid paraffin | 5.0 |
| | Hexalan (produced by Kyoei Kagaku Kogyo KK) | 3.0 |
| | Paraffin | 4.0 |
| | Beeswax HP (produced by Kyoei Kagaku Kogyo KK) | 4.0 |
| | Sorbitan monostearate | 1.0 |
| | Polyoxyethylene (5) sorbitan monostearate | 3.0 |
| | Glycerin monostearate | 1.0 |
| (B) | Glycerin | 4.0 |
| | L-ascorbic acid 2-phosphate zinc salt (Example 1) | 3.0 |
| | β-Cyclodextrin | 1.0 |
| | Water | 70.8 |
| (C) | Perfume | 0.2 |

Ingredient (A) and Ingredient (B) each was heated at 80°C, then mixed and stirred. After cooling to 50°C, Ingredient (C) was added, and the ingredients were mixed by further stirring to prepare a homogeneous cream.

Example 4 — Medical Preparations

(1) Preparation for an injectable drug (e.g., a drug for supplementing vitamin C)

| | |
|---|---|
| L-ascorbic acid 2-phosphate zinc salt (Example 1) | 1.0 wt% |
| glucose | 5 wt% |
| distilled water | 94 wt% |

The above ingredients were dissolved in distilled water and sterilized by filtering through a membrane filter with a pore size of 0.2 μm.

(2) Preparation for an oral capsule drug (e.g., an antiulcer drug)

| | |
|---|---|
| L-ascorbic acid 2-phosphate zinc salt (Example 1) | 10 wt% |
| corn starch | 85 wt% |
| magnesium stearate | 5 wt% |

The above ingredients were thoroughly mixed using a mill, and 100 mg of the mixture was inserted into a gelatin capsule.

(3) Preparation for an oral powder drug (e.g., an antiulcer drug)

| | |
|---|---|
| L-ascorbic acid 2-phosphate zinc salt (Example 1) | 10 wt% |
| crystalline lactose | 90 wt% |

The above ingredients were thoroughly mixed using a mill.

(4) Preparation for an ointment drug (e.g., a drug for acne treatment)

| | |
|---|---|
| L-ascorbic acid 2-phosphate zinc salt (Example 1) | 5 wt% |
| squalene | 10 wt% |
| stearic acid | 10 wt% |
| propyleneglycol monostearate | 3 wt% |
| polyoxyethylene cetyl ether | 1 wt% |
| propyleneglycol | 15 wt% |
| methyl p-hydroxybenzoate | 0.2 wt% |
| distilled water | 55.8 wt% |

The above ingredients were mixed and stirred at 80°C to obtain a homogeneous ointment.

<u>Example 5 — Feedstuff Preparations</u>

(1) Preparation for a feed additive for cattle, chicken, dogs and cats (e.g., a vitamin C supplement)

| | |
|---|---|
| L-ascorbic acid 2-phosphate zinc salt (Example 1) | 0.06 wt% |
| wheat bran | 30 wt% |
| barley meal | 20 wt% |
| rice bran | 42 wt% |
| soybean meal | 5 wt% |
| sodium chloride | 0.5 wt% |
| vitamin mixture (not containing vitamin C) | 2.44 wt% |

The above ingredients were mixed and pelleted using a pelletter at 80°C and dried at 100°C.

(2) Preparation for a feed additive for fish (e.g., a vitamin C supplement)

| | |
|---|---|
| L-ascorbic acid 2-phosphate zinc salt (Example 1) | 0.01 wt% |
| fish meal | 56 wt% |
| salmon milt | 6 wt% |
| squid liver oil | 5 wt% |
| soybean lecithin | 4 wt% |
| valine | 0.3 wt% |
| isoleucine | 0.2 wt% |
| corn starch | 5 wt% |
| mineral mixture | 5 wt% |
| gluten | 8 wt% |
| vitamin mixture (not containing vitamin C) | 2.59 wt% |
| ω3-HUFA | 1 wt% |
| soybean meal | 6.9 wt% |

The above ingredients were mixed and pelleted using a pelletter at 80°C and dried at 100°C.

Example 6 — Foodstuff Preparation

(1) Preparation for a food additive (e.g., a vitamin C supplement)

| | |
|---|---|
| L-ascorbic acid 2-phosphate zinc salt (Example 1) | 10 wt% |
| citric acid disodium salt | 0.5 wt% |
| oligosugar | 89.5 wt% |

The above ingredients were thoroughly mixed using a mill.

The L-ascorbic acid 2-phosphate zinc salt of the present invention is highly soluble and stable under weakly acidic conditions, and also has antimicrobial properties. By adding the salt to cosmetic preparations, medical preparations, agricultural chemical preparations, animal drug preparations, foodstuff preparations and feedstuff preparations, a vitamin C-containing preparation having improved solubility and stability and having an antimicrobial effect can be obtained.

## Claims

1. L-Ascorbic acid 2-phosphate zinc salt.

2. A salt hydrate of zinc L-ascorbic acid 2-phosphate.

3. The L-ascorbic acid 2-phosphate zinc salt as claimed in claim 1 or 2, wherein 95 mol% or more of the entire cationic charge of said salt constitutes zinc ion and 95 mol% or more of the entire anionic charge of said salt constitutes L-ascorbic acid 2-phosphate ion.

4. A process for manufacturing L-ascorbic acid 2-phosphate zinc salt, which comprises displacing a cation of a salt of an L-ascorbic acid 2-phosphate other than a zinc salt with a zinc cation.

5. The process for manufacturing L-ascorbic acid 2-phosphate zinc salt as claimed in claim 4, wherein 95 mol% or more of the entire cationic charge of said salt constitutes zinc ion and 95 mol% or more of the entire anionic charge of said salt constitutes L-ascorbic acid 2-phosphate ion.

6. A composition comprising L-ascorbic acid 2-phosphate zinc salt or a salt hydrate of zinc L-ascorbic acid 2-phosphate as an active ingredient.

7. The composition as claimed in claim 6, wherein 95 mol% or more of the entire cationic charge of said salt or salt hydrate constitutes zinc ion and 95 mol% or more of the entire anionic charge of said salt or salt hydrate constitutes L-ascorbic acid 2-phosphate ion.

8. The composition as claimed in claim 6, wherein said composition is selected from the group consisting of a cosmetic preparation, a medical preparation, an agricultural chemical preparation, an animal drug preparation, a foodstuff preparation and a feedstuff preparation.

9. A vitamin C agent comprising L-ascorbic acid 2-phosphate zinc salt or a salt hydrate of zinc L-ascorbic acid 2-phosphate as an active ingredient.

10. The composition as claimed in claim 6, containing L-ascorbic acid 2-phosphate zinc salt in an amount of from 0.01-50 wt% when the composition is a cosmetic preparation, a medical preparation, an agricultural chemical preparation or an animal drug preparation, or in an amount of from 0.001-50 wt% when the composition is a foodstuff preparation or a feedstuff preparation.

**Patentansprüche**

1. L-Ascorbinsäure-2-phosphatzinksalz.

2. Salzhydrat von Zink-L-Ascorbinsäure-2-phosphat.

3. L-Ascorbinsäure-2-phosphatzinksalz nach Anspruch 1 oder 2, worin Zinkionen 95 Mol-% oder mehr der gesamten kationischen Ladung des Salzes ausmachen und L-Ascorbinsäure-2-phosphationen 95 Mol-% oder mehr der gesamten anionischen Ladung des Salzes ausmachen.

4. Verfahren zur Herstellung von L-Ascorbinsäure-2-phosphatzinksalz, welches das Ersetzen eines Kations eines Salzes von L-Ascorbinsäure-2-phosphat, welches kein Zinksalz ist, durch ein Zinkkation umfasst.

5. Verfahren zur Herstellung von L-Ascorbinsäure-2-phosphatzinksalz nach Anspruch 4, worin Zinkionen 95 Mol-% oder mehr der gesamten kationischen Ladung des Salzes ausmachen und L-Ascorbinsäure-2-phosphationen 95 Mol-% oder mehr der gesamten anionischen Ladung des Salzes ausmachen.

6. Zusammensetzung, welche L-Ascorbinsäure-2-phosphatzinksalz oder ein Salzhydrat von Zink-L-Ascorbinsäure-2-phosphat als aktiven Bestandteil enthält.

7. Zusammensetzung nach Anspruch 6, worin Zinkionen 95 Mol-% oder mehr der gesamten kationischen Ladung des Salzes oder Salzhydrats ausmachen und L-Ascorbinsäure-2-phosphationen 95 Mol-% oder mehr der gesamten anionischen Ladung des Salzes oder Salzhydrats ausmachen.

8. Zusammensetzung nach Anspruch 6, worin die Zusammensetzung aus der Gruppe ausgewählt ist, die aus einer kosmetischen Zubereitung, einer medizinischen Zubereitung, einer landwirtschaftlich-chemischen Zubereitung, einer Tierarzneimittelzubereitung, einer Nahrungsmittelzubereitung und einer Futtermittelzubereitung besteht.

9. Vitamin-C-Mittel, welches L-Ascorbinsäure-2-phosphatzinksalz oder ein Salzhydrat von Zink-L-Ascorbinsäure-2-phosphat als aktiven Bestandteil enthält.

10. Zusammensetzung nach Anspruch 6, welche L-Ascorbinsäure-2-phosphatzinksalz in einer Menge von 0,01 bis 50 Gew.-%, wenn die Zusammensetzung eine kosmetische Zubereitung, eine medizinische Zubereitung, eine landwirtschaftlich-chemische Zubereitung oder eine Tierarzneimittelzubereitung ist, oder in einer Menge von 0,001 bis 50 Gew.-% enthält, wenn die Zusammensetzung eine Nahrungsmittelzubereitung oder eine Futtermittelzubereitung ist.

**Revendications**

1. Sel de zinc du 2-phosphate de l'acide L-ascorbique.

2. Hydrate du sel de zinc du 2-phosphate de l'acide L-ascorbique.

3. Sel de zinc du 2-phosphate de l'acide L-ascorbique selon la revendication 1 ou 2, où 95 % en mole ou plus de la totalité de la charge cationique dudit sel est constituée par l'ion zinc et 95 % en mole ou plus de la totalité de la charge anionique dudit sel est constituée par l'ion 2-phosphate de l'acide L-ascorbique.

4. Procédé pour la fabrication de sel de zinc du 2-phosphate de l'acide L-ascorbique, comprenant le déplacement du cation d'un sel du 2-phosphate de l'acide L-ascorbique autre qu'un sel de zinc par un cation zinc.

5. Procédé pour la fabrication de sel de zinc du 2-phosphate de l'acide L-ascorbique selon la revendication 4, où 95 % en mole ou plus de la totalité de la charge cationique dudit sel est constituée par l'ion zinc et 95 % en mole ou plus de la totalité de la charge anionique dudit sel est constituée par l'ion 2-phosphate de l'acide L-ascorbique.

6. Composition comprenant du sel de zinc du 2-phosphate de l'acide L-ascorbique ou un hydrate du sel de zinc du 2-phosphate de l'acide L-ascorbique en tant que principe actif.

7. Composition selon la revendication 6, où 95 % en mole ou plus de la totalité de la charge cationique dudit sel ou hydrate de sel est constituée par l'ion zinc et 95 % en mole ou plus de la totalité de la charge anionique dudit sel ou hydrate de sel est constituée par l'ion 2-phosphate de l'acide L-ascorbique.

8. Composition selon la revendication 6, ladite composition étant choisie dans le groupe constitué par une préparation cosmétique, une préparation médicale, une préparation chimique pour l'agriculture, une préparation de médicament vétérinaire, une préparation pour l'alimentation humaine et une préparation pour l'alimentation animale.

9. Agent vitaminique C comprenant le sel de zinc du 2-phosphate de l'acide L-ascorbique ou un hydrate du sel de zinc du 2-phosphate de l'acide L-ascorbique en tant que principe actif.

10. Composition selon la revendication 6, contenant une quantité de 0,01 à 50 % en masse de sel de zinc du 2-phosphate de l'acide L-ascorbique si la composition est une préparation cosmétique, une préparation médicale, une préparation chimique pour l'agriculture ou une préparation de médicament vétérinaire ou une quantité de 0,001 à 50 % en masse de ce sel si la composition est une préparation pour l'alimentation humaine ou une préparation pour l'alimentation animale.